# EUROPEAN PATENT APPLICATION

(11) **EP 3 854 396 A1**
(43) Date of publication of application: **28.07.2021**
(21) Application number: 19861634.4
(22) Date of filing: 18.09.2019
(51) Int. Cl.: A61K 31/4709, A61P 35/00

(54) **QUINOLINE DERIVATIVE USED FOR TREATING SMALL CELL LUNG CANCER**

(30) Priority: 18.09.2018 CN 201811088089
(71) Applicant: Chia Tai Tianqing Pharmaceutical Group Co., Ltd., Lianyungang, Jiangsu 222062 (CN)
(72) Inventor: WANG, Shanchun, Lianyungang, Jiangsu 222062 (CN); YU, Ding, Lianyungang, Jiangsu 222062 (CN); WANG, Xunqiang, Lianyungang, Jiangsu 222062 (CN); PENG, Bangan, Lianyungang, Jiangsu 222062 (CN); LIU, Lu, Lianyungang, Jiangsu 222062 (CN); DAI, Jie, Lianyungang, Jiangsu 222062 (CN); ZHOU, Hang, Lianyungang, Jiangsu 222062 (CN); MIAO, Yadong, Lianyungang, Jiangsu 222062 (CN)
(74) Representative: Geling, Andrea
(86) International application number: PCT/CN2019/106406
(87) International publication number: WO 2020/057539

(57) **Abstract**

The present application relates to the field of medicine, and provided thereby is a quinoline derivative used for treating small cell lung cancer. The 1-[[[4-(4-fluoro-2-methyl-1*H*-indol-5-yl)oxy-6-methoxyquinolin-7-yl]oxy] methyl]cyclopropylamine or a pharmaceutically acceptable salt thereof as provided by the present application may be used for the treatment of small cell lung cancer, especially for patients suffering from refractory relapsed small cell lung cancer, which may significantly prolong the survival time thereof; in addition, the therapeutic effect on brain metastatic small cell lung cancer is also very significant.

## Description

### CROSS-REFERENCE TO RELATED APPLICATIONS

The present application claims priority and benefit to the Chinese Patent Application No. 201811088089.6, filed with China National Intellectual Property Administration, on September 18, 2018, the disclosure of which is incorporated herein by reference in its entirety.

### TECHNICAL FIELD

The present application belongs to the field of pharmaceuticals, and particularly relates to use of a quinoline derivative in treating small cell lung cancer and a treatment method therefor, a pharmaceutical composition and a kit.

### BACKGROUND

Lung cancer is a malignant tumor with high incidence and mortality worldwide. In recent years, the incidence and mortality of lung cancer in China are rapidly increasing. The small cell lung cancer (SCLC), which accounts for 15%-20% of all lung cancer cases, is quite different from non-small cell lung cancer in terms of tissue origin, biological characteristics, response to treatment, prognosis and the like, so it is considered to be a systemic disease, and features high malignancy, strong invasiveness and easy metastasis. SCLC is highly invasive and has poor prognosis, with the 5-year survival rate being less than 5%, and the mean survival time of untreated patients being only 2-4 months.

Chemotherapy and radiotherapy are primary treatments for SCLC. Although the primary treatment for SCLC is very effective in the initial treatment, most SCLC relapses and metastases soon after treatment. Because of rapid progression, susceptibility to drug resistance, and poor prognosis, only a few patients have the opportunity to receive a third-line treatment. According to a study, only 20% of SCLC patients receive a third-line treatment. Whether a third-line treatment is beneficial is still inconclusive, and there is no standard treatment regimen for a third-line chemotherapy of SCLC. The treatment of relapsed SCLC, especially relapsed and refractory SCLC, becomes more difficult due to the occurrence of chemotherapy resistance. Therefore, there is an urgent need to find new treatment methods to treat relapsed SCLC.

### SUMMARY

In a first aspect, the present application provides a method for treating small cell lung cancer, comprising administering to a patient in need of treatment a therapeutically effective amount of a compound of formula I or a pharmaceutically acceptable salt thereof.

The chemical name of the compound of formula I is 1-[[[4-(4-fluoro-2-methyl-1*H*-indol-5-yl)oxy-6-methoxyquinolin-7-yl]oxy]methyl]cyclopropylamine, which has the following structural formula:

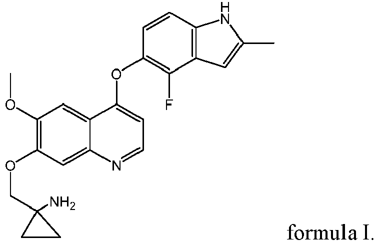

In a second aspect, the present application provides use of the compound of formula I or a pharmaceutically acceptable salt thereof in treating small cell lung cancer or for the manufacture of a medicament for treating small cell lung cancer.

In a third aspect, the present application provides a pharmaceutical composition for treating small cell lung cancer, comprising a compound of formula I or a pharmaceutically acceptable salt thereof, and at least one pharmaceutically acceptable carrier.

In a fourth aspect, the present application provides a kit for treating small cell lung cancer, comprising (a) at least one unit dose of a pharmaceutical composition comprising a compound of formula I or a pharmaceutically acceptable salt thereof and (b) instruction for treating small cell lung cancer.

In some embodiments, the small cell lung cancer is relapsed small cell lung cancer, including but not limited to relapsed and/or progressive small cell lung cancer after failure of treatment with chemotherapy and/or targeted drugs. In some embodiments, the small cell lung cancer is advanced small cell lung cancer. In some embodiments, the small cell lung cancer is one that has failed with at least two chemotherapy regimens. In some embodiments, the small cell lung cancer is one that has failed with second-line and/or higher-line chemotherapy. In some embodiments, the small cell lung cancer is metastatic small cell lung cancer, wherein metastatic lesions include, but are not limited to, lymph node, pleura, bone, brain, pericardium, adrenal gland, and liver metastases.

In one aspect, the present application provides a method for treating small cell lung cancer, comprising administering to a patient in need of treatment a therapeutically effective amount of a compound of formula I or a pharmaceutically acceptable salt thereof. In some embodiments, the present application provides a method for treating small cell lung cancer that has failed with at least two chemotherapy regimens, comprising administering to a patient in need of treatment a therapeutically effective amount of the compound of formula I or the pharmaceutically acceptable salt thereof. In some embodiments, the present application provides a method for treating small cell lung cancer that has failed with second-line and/or higher-line chemotherapy, comprising administering to a patient in need of treatment a therapeutically effective amount of the compound of formula I or the pharmaceutically acceptable salt thereof. In one embodiment, the present application provides a method for treating refractory and relapsed small cell lung cancer, comprising administering to a patient in need of treatment a therapeutically effective amount of the compound of formula I or the pharmaceutically acceptable salt thereof. The "refractory and relapsed small cell lung cancer" refers to small cell lung cancer which is not in remission by chemotherapy, and small cell lung cancer which responds to chemotherapy but progresses within 3 months after the end of the chemotherapy. The chemotherapy includes first-line chemotherapy and second-line chemotherapy, including but not limited to one or more of the anti-tumor chemotherapies such as podophyllum, alkylating agent, camptothecin, taxane, antimetabolite and anti-tumor antibiotic; illustrative examples include, but are not limited to, one or more of cisplatin, etoposide, carboplatin, nedaplatin, oxaliplatin, miriplatin, lobaplatin, irinotecan, topotecan, paclitaxel, docetaxel, temozolomide, vinorelbine, gemcitabine, cyclophosphamide, doxorubicin, vincristine, bendamustine, epirubicin, methotrexate, and amrubicin. It will be understood by those skilled in the art that a patient can also receive radiotherapy prior to, concurrently with, or subsequent to the chemotherapy.

In some embodiments, the small cell lung cancer is extensive-stage small cell lung cancer.

In some embodiments, the small cell lung cancer is metastatic small cell lung cancer, wherein metastatic lesions include, but are not limited to, lymph node, pleura, bone, pericardium, adrenal gland, liver, and brain metastases. In some embodiments, the small cell lung cancer is one with brain metastasis.

In some embodiments, the small cell lung cancer is refractory and relapsed small cell lung cancer that has been previously treated with second-line chemotherapy. The second-line chemotherapy includes, but is not limited to, one or more of cisplatin, carboplatin, nedaplatin, oxaliplatin, miriplatin, lobaplatin, irinotecan, topotecan, paclitaxel, docetaxel, temozolomide, vinorelbine, gemcitabine, cyclophosphamide, doxorubicin, vincristine, bendamustine, epirubicin, methotrexate and amrubicin.

In some embodiments, the small cell lung cancer is refractory and relapsed small cell lung cancer that has been previously treated with one or more of irinotecan, platinum, paclitaxel, and docetaxel.

In some embodiments, the small cell lung cancer is refractory and relapsed small cell lung cancer that has been previously treated with irinotecan and platinum (*e.g.*, including but not limited to cisplatin, carboplatin, nedaplatin, oxaliplatin, miriplatin, and lobaplatin).

In some embodiments, the present application provides a method for treating small cell lung cancer with brain metastasis, comprising administering to a patient in need of treatment a therapeutically effective amount of the compound of formula I or the pharmaceutically acceptable salt thereof.

The compound of formula I can be administered in its free base form, or in the form of its salt, hydrate, or prodrug that may convert *in vivo* into the free base form. For example, within the scope of the present application, the pharmaceutically acceptable salt of the compound of formula I can be generated from various organic and inorganic acids according to methods well known in the art.

In some embodiments, the compound of formula I or the pharmaceutically acceptable salt thereof is administered in the form of a hydrochloride salt. In some embodiments, the compound of formula I or the pharmaceutically acceptable salt thereof is administered in the form of a monohydrochloride salt. In some embodiments, the compound of formula I or the pharmaceutically acceptable salt thereof is administered in the form of a dihydrochloride salt. In some embodiments, the compound of formula I or the pharmaceutically acceptable salt thereof is administered in the form of a crystalline hydrochloride salt. In some embodiments, the compound of formula I or the pharmaceutically acceptable salt thereof is administered in the form of a crystalline dihydrochloride salt. In some embodiments, the compound of formula I or the pharmaceutically acceptable salt thereof is administered in the form of a maleate salt.

The compound of formula I or the pharmaceutically acceptable salt thereof can be administered via multiple routes of administration including, but not limited to, oral, parenteral, intraperitoneal, intravenous, intra-arterial, transdermal, sublingual, intramuscular, rectal, transbuccal, intranasal, inhalational, vaginal, intraocular, topical, subcutaneous, intralipid, intra-articular and intrathecal administrations. In some specific embodiments, the administration is performed orally.

The amount of the compound of formula I or the pharmaceutically acceptable salt thereof administered can be determined according to the severity of the disease, the response of the disease, any treatment-related toxicity, and the age and health of a patient. In some embodiments, the daily dose of the compound of formula I or the pharmaceutically acceptable salt thereof is 2 mg to 30 mg. In some embodiments, the daily dose of the compound of formula I or the pharmaceutically acceptable salt thereof is 6 mg to 20 mg. In some embodiments, the daily dose of the compound of formula I or the pharmaceutically acceptable salt thereof is 8 mg to 20 mg. In some embodiments, the daily dose of the compound of formula I or the pharmaceutically acceptable salt thereof is 8 mg to 16 mg. In some embodiments, the daily dose of the compound of formula I or the pharmaceutically acceptable salt thereof is 8 mg to 14 mg. In some specific embodiments, the daily dose of the compound of formula I or the pharmaceutically acceptable salt thereof is 8 mg. In some specific embodiments, the daily dose of the compound of formula I or the pharmaceutically acceptable salt thereof is 10 mg. In some specific embodiments, the daily dose of the compound of formula I or the pharmaceutically acceptable salt thereof is 12 mg. In some specific embodiments, the daily dose of the compound of formula I or the pharmaceutically acceptable salt thereof is 14 mg.

The compound of formula I or the pharmaceutically acceptable salt thereof can be administered once or multiple times daily. In some embodiments, the compound of formula I or the pharmaceutically acceptable salt thereof is administered once daily. In some embodiments, the compound of formula I or the pharmaceutically acceptable salt thereof is administered once daily in the form of a solid oral preparation.

The dosage regimen can be determined comprehensively depending on the activity and toxicity of the medicament, tolerance of a patient, *etc.* Preferably, the compound of formula I or the pharmaceutically acceptable salt thereof is administered in an intermittent regimen. The intermittent regimen includes treatment periods and interruption periods. In the treatment period, the compound of formula I or the pharmaceutically acceptable salt thereof can be administered once or multiple times daily. For example, the compound of formula I or the pharmaceutically acceptable salt thereof is administered daily in the treatment period, and then the treatment is interrupted during the interruption period, followed by the treatment period and then the interruption period, over and over again. The ratio of the treatment period to the interruption period in days is 2:0.5-2:5, preferably 2:0.5-2:3, more preferably 2:0.5-2:2, and even more preferably 2:0.5-2:1.

In some embodiments, the drug is administered for 2 weeks and interrupted for 2 weeks. In some embodiments, the drug is administered once daily for 14 days and then interrupted for 14 days.

In some embodiments, the drug is administered for 2 weeks and interrupted for 1 week. In some embodiments, the treatment is administered once daily for 14 days and then interrupted for 7 days.

In some embodiments, the drug is administered for 5 days and interrupted for 2 days. In some embodiments, the drug is administered once daily for 5 days and then interrupted for 2 days.

In certain specific embodiments, the drug is administered once daily at a dose of 12 mg for 2 weeks and interrupted for 1 week.

In certain specific embodiments, the drug is administered once daily at a dose of 10 mg for 2 weeks and interrupted for 1 week.

In certain specific embodiments, the drug is administered once daily at a dose of 8 mg for 2 weeks and interrupted for 1 week.

In some embodiments, the compound of formula I or the pharmaceutically acceptable salt thereof is administered alone to a small cell lung cancer patient as the only active ingredient. In some embodiments, the compound of formula I or the pharmaceutically acceptable salt thereof is administered to a small cell lung cancer patient concurrently or sequentially with other anti-tumor drugs. In some embodiments, the other anti-tumor drugs include, but are not limited to, one or more of antifolates, podophyllums, hormones, alkylating agents, camptothecins, fluoropyrimidine derivatives, anthracyclines, taxanes, tyrosine kinase inhibitors (e.g., EGFR inhibitor and VEGFR inhibitor), mitomycin, and trastuzumab.

In a second aspect, the present application provides use of a compound of formula I or a pharmaceutically acceptable salt thereof in treating small cell lung cancer or for the manufacture of a medicament for treating small cell lung cancer. In some embodiments, the present application provides use of the compound of formula I or the pharmaceutically acceptable salt thereof in treating small cell lung cancer or for the manufacture of a medicament for treating small cell lung cancer that has failed with at least two chemotherapy regimens. In some embodiments, the present application provides use of the compound of formula I or the pharmaceutically acceptable salt thereof in treating small cell lung cancer or for the manufacture of a medicament for treating small cell lung cancer that has failed with second-line and/or higher-line chemotherapy. In one embodiment, the present application provides use of the compound of formula I or the pharmaceutically acceptable salt thereof in treating refractory and relapsed small cell lung cancer or for the manufacture of a medicament for treating refractory and relapsed small cell lung cancer.

The "refractory and relapsed small cell lung cancer" refers to small cell lung cancer which is not in remission by chemotherapy and small cell lung cancer which responds to chemotherapy but progresses within 3 months after the end of the chemotherapy. The chemotherapy includes first-line chemotherapy and second-line chemotherapy, including but not limited to one or more of the anti-tumor chemotherapies such as podophyllum, alkylating agent, camptothecin, taxane, antimetabolite and anti-tumor antibiotic; illustrative examples include, but are not limited to, one or more of cisplatin, etoposide, carboplatin, nedaplatin, oxaliplatin, miriplatin, lobaplatin, irinotecan, topotecan, paclitaxel, docetaxel, temozolomide, vinorelbine, gemcitabine, cyclophosphamide, doxorubicin, vincristine, bendamustine, epirubicin, methotrexate, and amrubicin. It will be understood by those skilled in the art that a patient can also receive radiotherapy and/or monoclonal antibody (*e.g.*, nivolumab and ipilimumab) therapy prior to, concurrently with, or subsequent to the chemotherapy.

In some embodiments, the small cell lung cancer is extensive-stage small cell lung cancer.

In some embodiments, the small cell lung cancer is metastatic small cell lung cancer, wherein metastatic lesions include, but are not limited to, lymph node, pleura, bones, pericardium, adrenal glands, liver, and brain metastases. In some embodiments, the small cell lung cancer is one with brain metastasis.

In some embodiments, the small cell lung cancer is refractory and relapsed small cell lung cancer that has been previously treated with second-line chemotherapy. The second-line chemotherapy includes, but is not limited to, one or more of cisplatin, carboplatin, nedaplatin, oxaliplatin, miriplatin, lobaplatin, irinotecan, topotecan, paclitaxel, docetaxel, temozolomide, vinorelbine, gemcitabine, cyclophosphamide, doxorubicin, vincristine, bendamustine, epirubicin, methotrexate, and amrubicin.

In some embodiments, the small cell lung cancer is refractory and relapsed small cell lung cancer that has been previously treated with one or more of irinotecan, platinum, paclitaxel, and docetaxel.

In some embodiments, the small cell lung cancer is refractory and relapsed small cell lung cancer that has been previously treated with irinotecan and platinum (e.g., including but not limited to cisplatin, carboplatin, nedaplatin, oxaliplatin, miriplatin, and lobaplatin).

In some embodiments, the present application provides use of the compound of formula I or the pharmaceutically acceptable salt thereof in treating or for the manufacture of a medicament for treating small cell lung cancer with brain metastasis.

The compound of formula I can be administered in its free base form, or in the form of its salt, hydrate, or prodrug that may convert *in vivo* into the free base form. For example, within the scope of the present application, the pharmaceutically acceptable salt of the compound of formula I can be generated from various organic and inorganic acids according to methods well known in the art.

In some embodiments, the compound of formula I or the pharmaceutically acceptable salt thereof is in the form of a hydrochloride salt. In some embodiments, the compound of formula I or the pharmaceutically acceptable salt thereof is in the form of a monohydrochloride salt. In some embodiments, the compound of formula I or the pharmaceutically acceptable salt thereof is in the form of a dihydrochloride salt. In some embodiments, the compound of formula I or the pharmaceutically acceptable salt thereof is in the form of a crystalline hydrochloride salt. In specific embodiments, the compound of formula I or the pharmaceutically acceptable salt thereof is in the form of a crystalline dihydrochloride salt. In some embodiments, the compound of formula I or the pharmaceutically acceptable salt thereof is in the form of a maleate salt.

The compound of formula I or the pharmaceutically acceptable salt thereof can be administered via multiple routes of administration including, but not limited to, oral, parenteral, intraperitoneal, intravenous, intra-arterial, transdermal, sublingual, intramuscular, rectal, transbuccal, intranasal, inhalational, vaginal, intraocular, topical, subcutaneous, intralipid, intra-articular and intrathecal administrations. In some specific embodiments, the administration is performed orally.

The amount of the compound of formula I or the pharmaceutically acceptable salt thereof can be determined according to the severity of the disease, the response of the disease, any treatment-related toxicity, and the age and health of a patient. In some embodiments, the daily dose of the compound of formula I or the pharmaceutically acceptable salt thereof is 2 mg to 30 mg. In some embodiments, the daily dose of the compound of formula I or the pharmaceutically acceptable salt thereof is 6 mg to 20 mg. In some embodiments, the daily dose of the compound of formula I or the pharmaceutically acceptable salt thereof is 8 mg to 20 mg. In some embodiments, the daily dose of the compound of formula I or the pharmaceutically acceptable salt thereof is 8 mg to 16 mg. In some embodiments, the daily dose of the compound of formula I or the pharmaceutically acceptable salt thereof is 8 mg to 14 mg. In some specific embodiments, the daily dose of the compound of formula I or the pharmaceutically acceptable salt thereof is 8 mg. In some specific embodiments, the daily dose of the compound of formula I or the pharmaceutically acceptable salt thereof is 10 mg. In some specific embodiments, the daily dose of the compound of formula I or the pharmaceutically acceptable salt thereof is 12 mg. In some specific embodiments, the daily dose of the compound of formula I or the pharmaceutically acceptable salt thereof is 14 mg.

The compound of formula I or the pharmaceutically acceptable salt thereof can be administered once or multiple times daily. In some embodiments, the compound of formula I or the pharmaceutically acceptable salt thereof is administered once daily. In some embodiments, the compound of formula I or the pharmaceutically acceptable salt thereof is administered once daily in the form of a solid oral preparation.

The dosage regimen can be determined comprehensively depending on the activity and toxicity of the medicament, tolerance of a patient, *etc.* Preferably, the compound of formula I or the pharmaceutically acceptable salt thereof is administered in an intermittent regimen. The intermittent regimen includes treatment periods and interruption periods. In the treatment period, the compound of formula I or the pharmaceutically acceptable salt thereof can be administered once or multiple times daily. For example, the compound of formula I or the pharmaceutically acceptable salt thereof is administered daily in the treatment period, and then the treatment is interrupted during the interruption period, followed by the treatment period and then the interruption period, over and over again. The ratio of the treatment period to the interruption period in days is 2:0.5-2:5, preferably 2:0.5-2:3, more preferably 2:0.5-2:2, and even more preferably 2:0.5-2:1.

In some embodiments, the drug is administered for 2 weeks and interrupted for 2 weeks. In some embodiments, the drug is administered once daily for 14 days and then interrupted for 14 days.

In some embodiments, the drug is administered for 2 weeks and interrupted for 1 week. In some embodiments, the drug is administered once daily for 14 days and then interrupted for 7 days.

In some embodiments, the drug is administered for 5 days and interrupted for 2 days. In some embodiments, the drug is administered once daily for 5 days and then interrupted for 2 days.

In certain specific embodiments, the drug is administered once daily at a dose of 12 mg for 2 weeks and interrupted for 1 week.

In certain specific embodiments, the drug is administered once daily at a dose of 10 mg for 2 weeks and interrupted for 1 week.

In certain specific embodiments, the drug is administered once daily at a dose of 8 mg for 2 weeks and interrupted for 1 week.

In some embodiments, the compound of formula I or the pharmaceutically acceptable salt thereof is administered alone to a small cell lung cancer patient as the only active ingredient. In some embodiments, the compound of formula I or the pharmaceutically acceptable salt thereof is administered to a small cell lung cancer patient concurrently or sequentially with other anti-tumor drugs. In some embodiments, the other anti-tumor drugs include, but are not limited to, one or more of antifolates, podophyllums, hormones, alkylating agents, camptothecins, fluoropyrimidine derivatives, anthracyclines, taxanes, tyrosine kinase inhibitors (*e.g.*, EGFR inhibitor and VEGFR inhibitor), mitomycin, and trastuzumab.

In a third aspect, the present application provides a pharmaceutical composition for treating small cell lung cancer, comprising a compound of formula I or a pharmaceutically acceptable salt thereof, and at least one pharmaceutically acceptable carrier. In some embodiments, the present application provides a pharmaceutical composition for treating small cell lung cancer that has failed with at least two chemotherapy regimens, comprising the compound of formula I or the pharmaceutically acceptable salt thereof, and at least one pharmaceutically acceptable carrier. In some embodiments, the present application provides a pharmaceutical composition for treating small cell lung cancer that has failed with second-line and/or higher-line chemotherapy, comprising the compound of formula I or the pharmaceutically acceptable salt thereof, and at least one pharmaceutically acceptable carrier. In one embodiment, the present application provides a pharmaceutical composition for treating refractory and relapsed small cell lung cancer, comprising the compound of formula I or the pharmaceutically acceptable salt thereof, and at least one pharmaceutically acceptable carrier.

The "refractory and relapsed small cell lung cancer" refers to small cell lung cancer which is not in remission by chemotherapy and small cell lung cancer which responds to chemotherapy but progresses within 3 months after the end of the chemotherapy. The chemotherapy includes first-line chemotherapy and second-line chemotherapy, including but not limited to one or more of the anti-tumor chemotherapies such as podophyllum, alkylating agent, camptothecin, taxane, antimetabolite and anti-tumor antibiotic; illustrative examples include, but are not limited to, one or more of cisplatin, etoposide, carboplatin, nedaplatin, oxaliplatin, miriplatin, lobaplatin, irinotecan, topotecan, paclitaxel, docetaxel, temozolomide, vinorelbine, gemcitabine, cyclophosphamide, doxorubicin, vincristine, bendamustine, epirubicin, methotrexate, and amrubicin. It will be understood by those skilled in the art that a patient can also receive radiotherapy and/or monoclonal antibody (*e.g.*, nivolumab and ipilimumab) therapy prior to, concurrently with, or subsequent to the chemotherapy.

In some embodiments, the small cell lung cancer is extensive-stage small cell lung cancer.

In some embodiments, the small cell lung cancer is metastatic small cell lung cancer, wherein metastatic lesions include, but are not limited to, lymph node, pleura, bones, pericardium, adrenal glands, liver, and brain metastases. In some embodiments, the small cell lung cancer is one with brain metastasis.

In some embodiments, the small cell lung cancer is refractory and relapsed small cell lung cancer that has been previously treated with second-line chemotherapy.

In some embodiments, the small cell lung cancer is refractory and relapsed small cell lung cancer that has been previously treated with one or more of irinotecan, platinum, paclitaxel, and docetaxel.

In some embodiments, the small cell lung cancer is refractory and relapsed small cell lung cancer that has been previously treated with irinotecan and platinum (*e.g.*, including but not limited to cisplatin, carboplatin, nedaplatin, oxaliplatin, miriplatin, and lobaplatin).

In some embodiments, a pharmaceutical composition for treating small cell lung cancer with brain metastasis is provided, comprising the compound of formula I or the pharmaceutically acceptable salt thereof, and at least one pharmaceutically acceptable carrier.

The compound of formula I can be administered in its free base form, or in the form of its salt, hydrate, or prodrug that may convert *in vivo* into the free base form. For example, within the scope of the present application, the pharmaceutically acceptable salt of the compound of formula I can be generated from various organic and inorganic acids according to methods well known in the art.

In some embodiments, the compound of formula I or the pharmaceutically acceptable salt thereof is in the form of a hydrochloride salt. In some embodiments, the compound of formula I or the pharmaceutically acceptable salt thereof is in the form of a monohydrochloride salt. In some embodiments, the compound of formula I or the pharmaceutically acceptable salt thereof is in the form of a dihydrochloride salt. In some embodiments, the compound of formula I or the pharmaceutically acceptable salt thereof is in the form of a crystalline hydrochloride salt. In specific embodiments, the compound of formula I or the pharmaceutically acceptable salt thereof is in the form of a crystalline dihydrochloride salt. In some embodiments, the compound of formula I or the pharmaceutically acceptable salt thereof is in the form of a maleate salt.

In some embodiments, the pharmaceutical composition may be a preparation suitable for oral, parenteral, intraperitoneal, intravenous, intra-arterial, transdermal, sublingual, intramuscular, rectal, transbuccal, intranasal, inhalational, vaginal, intraocular, topical, subcutaneous, intralipid, intra-articular and intrathecal administrations. In some embodiments, the amount of the pharmaceutical composition administered can be determined according to the severity of the disease, the response of the disease, any treatment-related toxicity, and the age and health of a patient. In some embodiments, the daily dose of the compound of formula I or the pharmaceutically acceptable salt thereof is 2 mg to 30 mg. In some embodiments, the daily dose of the compound of formula I or the pharmaceutically acceptable salt thereof is 6 mg to 20 mg. In some embodiments, the daily dose of the compound of formula I or the pharmaceutically acceptable salt thereof in the pharmaceutical composition is 8 mg to 20 mg. In some embodiments, the daily dose of the compound of formula I or the pharmaceutically acceptable salt thereof is 8 mg to 16 mg. In some embodiments, the daily dose of the compound of formula I or the pharmaceutically acceptable salt thereof is 8 mg to 14 mg. In some specific embodiments, the daily dose of the compound of formula I or the pharmaceutically acceptable salt thereof is 8 mg. In some specific embodiments, the daily dose of the compound of formula I or the pharmaceutically acceptable salt thereof is 10 mg. In some specific embodiments, the daily dose of the compound of formula I or the pharmaceutically acceptable salt thereof is 12 mg. In some specific embodiments, the daily dose of the compound of formula I or the pharmaceutically acceptable salt thereof is 14 mg.

In some embodiments of the present application, the pharmaceutical composition is a preparation suitable for oral administration, including tablets, capsules, powders, granules, dripping pills, pastes, pulvis, and the like, preferably tablets and capsules. The tablet may be a common tablet, dispersible tablet, effervescent tablet, sustained-release tablet, controlled-release tablet or enteric coated tablet. The capsule may be a common capsule, sustained-release capsule, controlled-release capsule or enteric coated capsule. The oral preparation may be prepared by a conventional method using a pharmaceutically acceptable carrier well known in the art. The pharmaceutically acceptable carrier includes fillers, absorbents, wetting agents, binders, disintegrants, lubricants, and the like. The fillers include starch, lactose, mannitol, microcrystalline cellulose, and the like. The absorbents include calcium sulfate, calcium hydrogen phosphate, calcium carbonate, and the like. The wetting agents include water, ethanol, and the like. The binders include hydroxypropyl methylcellulose, polyvidone, microcrystalline cellulose, and the like. The disintegrants include croscarmellose sodium, crospovidone, surfactants, low-substituted hydroxypropyl cellulose, and the like. The lubricants include magnesium stearate, talcum powder, polyethylene glycol, sodium dodecyl sulfate, colloidal silicon dioxide, talcum powder, and the like. The pharmaceutically acceptable carrier further includes coloring agents, sweeteners and the like.

In some embodiments, the pharmaceutical composition is a solid preparation suitable for oral administration. The composition, for example, may be in the form of a tablet or capsule. In some specific embodiments, the pharmaceutical composition is a capsule. In some specific embodiments of the present application, the pharmaceutically acceptable carrier of the solid oral preparation includes mannitol, microcrystalline cellulose, hydroxypropylcellulose, and magnesium stearate.

The compound of formula I or the pharmaceutically acceptable salt thereof can be administered once or multiple times daily. In some embodiments, the compound of formula I or the pharmaceutically acceptable salt thereof is administered once daily. In some embodiments, the compound of formula I or the pharmaceutically acceptable salt thereof is administered once daily in the form of a solid oral preparation.

The dosage regimen can be determined comprehensively depending on the activity and toxicity of the medicament, tolerance of a patient, *etc.* Preferably, the compound of formula I or the pharmaceutically acceptable salt thereof is administered in an intermittent regimen. The intermittent regimen includes treatment periods and interruption periods. In the treatment period, the compound of formula I or the pharmaceutically acceptable salt thereof can be administered once or multiple times daily. For example, the compound of formula I or the pharmaceutically acceptable salt thereof is administered daily in the treatment period, and then the treatment is interrupted during the interruption period, followed by the treatment period and then the interruption period, over and over again. The ratio of the treatment period to the interruption period in days is 2:0.5-2:5, preferably 2:0.5-2:3, more preferably 2:0.5-2:2, and even more preferably 2:0.5-2:1.

In some embodiments, the drug is administered for 2 weeks and interrupted for 2 weeks. In some embodiments, the drug is administered once daily for 14 days and then interrupted for 14 days.

In some embodiments, the drug is administered for 2 weeks and interrupted for 1 week. In some embodiments, the treatment is administered once daily for 14 days and then interrupted for 7 days.

In some embodiments, the drug is administered for 5 days and interrupted for 2 days. In some embodiments, the treatment is administered once daily for 5 days and then interrupted for 2 days.

In certain specific embodiments, the drug is administered once daily at a dose of 12 mg for 2 weeks and interrupted for 1 week.

In certain specific embodiments, the drug is administered once daily at a dose of 10 mg for 2 weeks and interrupted for 1 week.

In certain specific embodiments, the drug is administered once daily at a dose of 8 mg for 2 weeks and interrupted for 1 week.

In some embodiments, a pharmaceutical composition in unit dosage form for treating refractory and relapsed small cell lung cancer is provided. In some embodiments, the pharmaceutical composition in unit dosage form comprises 2 mg to 30 mg of the compound of formula I or the pharmaceutically acceptable salt thereof. In some embodiments, the pharmaceutical composition in unit dosage form comprises 6 mg to 20 mg of the compound of formula I or the pharmaceutically acceptable salt thereof. In some embodiments, the pharmaceutical composition in unit dosage form comprises 8 mg to 20 mg, preferably 8 mg to 16 mg, and more preferably 8 mg to 14 mg of the compound of formula I or the pharmaceutically acceptable salt thereof. In some specific embodiments, the pharmaceutical composition in unit dosage form comprises 8 mg of the compound of formula I or the pharmaceutically acceptable salt thereof. In some specific embodiments, the pharmaceutical composition in unit dosage form comprises 10 mg of the compound of formula I or the pharmaceutically acceptable salt thereof. In some specific embodiments, the pharmaceutical composition in unit dosage form comprises 12 mg of the compound of formula I or the pharmaceutically acceptable salt thereof. In some specific embodiments, the pharmaceutical composition in unit dosage form comprises 14 mg of the compound of formula I or the pharmaceutically acceptable salt thereof.

In some embodiments, the compound of formula I or the pharmaceutically acceptable salt thereof is administered alone to a patient with relapsed small cell lung cancer as the only active ingredient. In some embodiments, the compound of formula I or the pharmaceutically acceptable salt thereof is administered to a patient with relapsed small cell lung cancer concurrently or sequentially with other anti-tumor drugs. In some embodiments, the other anti-tumor drugs include, but are not limited to, one or more of antifolates, podophyllums, hormones, alkylating agents, camptothecins, fluoropyrimidine derivatives, anthracyclines, taxanes, tyrosine kinase inhibitors (e.g., EGFR inhibitor and VEGFR inhibitor), mitomycin, and trastuzumab.

In a fourth aspect, the present application provides a kit, comprising (a) at least one unit dose of the pharmaceutical composition comprising the compound of formula I or the pharmaceutically acceptable salt thereof and (b) instruction for treating small cell lung cancer. In some embodiments, the present application provides a kit, comprising (a) at least one unit dose of the pharmaceutical composition comprising the compound of formula I or the pharmaceutically acceptable salt thereof and (b) instruction for treating small cell lung cancer that has failed with at least two chemotherapy regimens. In some embodiments, the present application provides a kit, comprising (a) at least one unit dose of the pharmaceutical composition comprising the compound of formula I or the pharmaceutically acceptable salt thereof and (b) instruction for treating small cell lung cancer that has failed with second-line and/or higher-line chemotherapy. In one embodiment, the present application provides a kit, comprising (a) at least one unit dose of the pharmaceutical composition comprising the compound of formula I or the pharmaceutically acceptable salt thereof and (b) instruction for treating refractory and relapsed small cell lung cancer.

The present application also provides a kit, comprising (a) at least one unit dose of a preparation suitable for oral administration comprising the compound of formula I or the pharmaceutically acceptable salt thereof and (b) instruction for treating refractory and relapsed small cell lung cancer in an intermittent regimen. In some specific embodiments, a kit is provided, comprising (a) at least one unit dose of a tablet or capsule comprising the compound of formula I or the pharmaceutically acceptable salt thereof and (b) instruction for treating refractory and relapsed small cell lung cancer in an intermittent regimen.

Herein, it will be understood by those skilled in the art that a patient in need of treatment can receive radiotherapy and chemotherapy alternatively, sequentially or currently with the administration of a therapeutically effective amount of the compound of formula I or the pharmaceutically acceptable salt thereof.

Herein, the "unit dose" or "unit dosage" refers to a pharmaceutical composition contained in a single package, such as each tablet or capsule, for ease of administration. For example, for tablets or capsules, "pharmaceutical composition in unit dosage form" refers to each tablet or capsule.

Herein, unless otherwise stated, the dose and ranges are based on the molecular weight of the free base form for the compound of formula I.

Herein, the crystalline hydrochloride salt of the compound of formula I includes, but is not limited to, crystalline form A, crystalline form B and crystalline form C disclosed in Chinese Patent Application No. CN102344438A. The crystalline form A and crystalline form B are substantially free of crystalline water and other solvents, and crystalline form C contains two crystalline water. In some embodiments, the crystalline dihydrochloride salt of the compound of formula I is crystalline form A.

Unless otherwise stated, the following terms used in the specification and claims shall have the following meanings for the purposes of the present invention.

"Patient" refers to a mammal, and preferably a human.

"Pharmaceutically acceptable" refers to that when a substance is used for preparing a pharmaceutical composition, the pharmaceutical composition is generally safe, non-toxic, and desirable biologically and otherwise, and inclusion of the substance is acceptable for pharmaceutical use in human.

"Pharmaceutically acceptable salt" includes, but is not limited to, acid addition salts of inorganic acids such as hydrochloric acid, hydrobromic acid, sulfuric acid, nitric acid and phosphoric acid; or acid addition salts of organic acids such as acetic acid, trifluoroacetic acid, propionic acid, hexanoic acid, heptanoic acid, cyclopentane propionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, 1,2-ethanedisulfonic acid, 2-hydroxyethanesulfonic acid, benzenesulfonic acid, *p*-chlorobenzenesulfonic acid, *p*-toluenesulfonic acid, 3-phenylpropionic acid, trimethylacetic acid, *t*-butylacetic acid, dodecyl sulfuric acid, gluconic acid, glutamic acid, hydroxynaphthoic acid, salicylic acid and stearic acid.

"Therapeutically effective amount" refers to an amount of a compound that, when administered to a human for treating a disease, is sufficient to treat the disease.

"Therapy" or "treatment" or "treat" refers to any administration of a therapeutically effective amount of a compound, and is intended to:
(1) suppress a disease in a human experiencing or exhibiting the pathology or symptomatology of the disease (*i.e.,* preventing further pathological and/or symptomatological progression), or
(2) relieve the disease in a human experiencing or exhibiting the pathology or symptomatology of the disease (*i.e.,* reversing the pathology and/or symptomatology).

"Treatment failure" or "fail with treatment" refers to the intolerance of toxicity and side effects, disease progression during treatment, or relapse after treatment.

"Extended stage" means that lesions extend beyond one side of the thoracic cavity and include malignant pericardial effusion or hematogenous metastasis.

"EGFR inhibitor" refers to epidermal growth factor receptor inhibitor.

"VEGFR inhibitor" refers to vascular endothelial growth factor receptor inhibitor.

"CR" refers to complete response, where all target lesions disappear, and the short-axis diameter of all pathological lymph nodes (including target and non-target nodes) must be reduced to < 10 mm.

"PR" refers to partial response, where the sum of the diameters of target lesions is reduced by at least 30% from the baseline level.

"PD" refers to progressive disease, where the sum of the diameters is relatively increased by at least 20% with reference to the minimum of the sum of the measured diameters of all target lesions throughout the study (or with reference to the baseline if the baseline measurement is minimal); and the relative value of the sum of the diameters must be increased by at least 5 mm. The presence of one or more new lesions is also considered as PD.

"SD" refers to stable disease, where the target lesions are neither sufficiently reduced to level of PR nor sufficiently increased to level of PD, somewhere in between.

"PFS" refers to progression-free survival, *i.e.,* the time from randomization until objective progression of the tumor or death of a patient.

"DCR" refers to disease control rate, including the percentage of cases with complete response, partial response, and stable disease maintained for more than 4 weeks among patients suitable for efficacy evaluation.

"OS" refers to overall survival, *i.e.,* the time from the start of enrollment to death due to any cause, calculated by days. For subjects who are lost to follow-up, the time of the last follow-up is typically regarded as the death time. "FAS" refers to full analysis set, including all cases using a medicament at least once that are analyzed for efficacy in accordance with the principle of intention-to-treat (ITT).

"RECIST 1.1" refers to response evaluation criteria in solid tumor 1.1.

"CI" refers to confidence interval, *i.e.,* the estimated interval of the population parameter constructed by the sample statistics.

The compound of formula I or the pharmaceutically acceptable salt thereof disclosed herein has a surprising therapeutic effect on small cell lung cancer, especially on refractory and relapsed small cell lung cancer. It can remarkably prolong the survival time of a patient with refractory and relapsed small cell lung cancer, and also has a remarkable therapeutic effect on small cell lung cancer with brain metastasis.

### DETAILED DESCRIPTION

The present application is further illustrated below with reference to specific examples. It should be understood that these examples are only for illustrative purposes and not intended to limit the scope of the present application. **Example 1** 1-[[[4-(4-fluoro-2-methyl-1*H*-indol-5-yl)oxy-6-methoxyquinolin-7-yl]oxy]methyl]cyclopropylamine dihydrochloride (dihydrochloride of the compound of formula I)

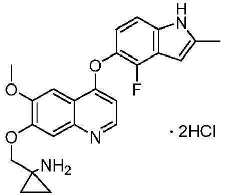

1-[[[4-(4-fluoro-2-methyl-1*H*-indol-5-yl)oxy-6-methoxyquinolin-7-yl]oxy]methyl]cyclopropylamine was prepared referring to the Example 24 of WO2008112407, and then the titled compound was prepared referring to the preparation method of "Examples of salt formation" in the specification of WO2008112407. Alternatively, the titled compound may be prepared referring to the method disclosed in Chinese Patent Application No. CN10234443 8A.

### Example 2 Capsule containing 1-[[[4-(4-fluoro-2-methyl-1H-indol-5-yl)oxy-6-methoxyquinolin-7-yl]oxy] methyl]cyclopropylamine dihydrochloride (dihydrochloride of the compound of formula I)

| | |
|---|---|
| API and excipients | dosage (1000 capsules) |
| Dihydrochloride of the compound of formula I | 14.16 g (equivalent to 12 g of the compound of formula I) |
| Mannitol | 89 g |
| Microcrystalline cellulose | 138.4 g |
| Hydroxypropyl cellulose | 5.9 g |
| Magnesium stearate | 0.99 g |

The dihydrochloride of the compound of formula I was ground, sieved with an 80-mesh sieve, and well mixed with mannitol and hydroxypropyl cellulose. A prescribed amount of microcrystalline cellulose was added, and the resulting mixture was well mixed and sieved with a 0.8-mm sieve. Finally, a prescribed amount of magnesium stearate was added and the resulting mixture was well mixed to fill capsules.

Capsules with different amount of the dihydrochloride of the compound of formula I can be prepared according to the corresponding proportions and formulation as described above.

### Example 3

A multicenter, randomized, double-blind and placebo-controlled phase II clinical study was conducted for patients with relapsed small cell lung cancer characterized by presence of measurable lesions and prior standard treatment. In this study, 120 patients were enrolled and randomized to receive oral administration of 1 capsule of the dihydrochloride of the compound of formula I at 12 mg/0 mg once daily. The oral administration was continued for 2 weeks and interrupted for 1 week, *i.e.,* 1 treatment cycle took 3 weeks (21 days). These patients aged 18-75 were treated with the capsule of the dihydrochloride of the compound of formula I and placebo control, and patients who had previously used other targeted drugs (such as sunitinib, bevacizumab, and endostar) and immunotargeted drugs were excluded. In the case of progressive disease (PD) determined by the response evaluation criteria or judged clinically, treatment with other anti-tumor drugs (such as chemotherapy, targeted therapy or biological agent therapy) influencing the judgment of efficacy, unexpected pregnancy, death and the like, the administration was interrupted (terminated) for a subject. For patients with disease under control (CR + PR + SD) and tolerable adverse reactions, the administration was continued. For patients who were unsuitable for continued administration judged by the investigator or were evaluated as PD according to RECIST 1.1 criteria, the administration was terminated. During the administration, depending on the drug-related toxicity degree and the possible efficacy benefit of a patient, the investigator determined whether to adjust the dosage, and the adjustable dosage was 8 mg/0 mg or 10 mg/0 mg.

The primary efficacy endpoint was progression-free survival (PFS), and the secondary efficacy endpoints were overall survival (OS), objective response rate (ORR) (CR + PR), disease control rate (DCR) (CR + PR + SD), quality of life score and safety. The objective efficacy endpoint was evaluated according to the response evaluation criteria in solid tumor (RECIST 1.1).

All statistical analyses were conducted using the SAS 9.2 statistical analysis software programming. All statistical tests were conducted using bilateral test. When *P* value ≤ 0.05, the tested difference was considered statistically significant, with the confidence interval being 95% confidence.

For the primary efficacy endpoint PFS, 50% (median) PFS and PFS at different time points after the start of the treatment were calculated by using the product limit method according to the data situation, and the two groups were compared using Log-Rank test. For OS, 50% (median) OS was calculated by using the product limit method according to the data situation, and the two groups were compared using Log-Rank test. The full analysis set (FAS) was used for the statistical dataset. The results of the study are summarized in the following table.

**Table 1. Progression-free survival of patients (PFS, month)**

| | Test group | Control group | *P* value |
|---|---|---|---|
| n | 81 | 38 | <0.0001 |
| Number of censored cases (%) | 28(34.57) | 5(13.16) | |
| Median (95%CI) | 4.07(2.79, 4.24) | 0.72(0.69, 0.82) | |

**Table 2. Overall survival of patients (OS, month**

| | Test group | Control group | *P* value |
|---|---|---|---|
| n | 81 | 38 | 0.0210 |
| Number of censored cases (%) | 49(60.49) | 17(44.74) | |
| Median (95%CI) | 7.29(6.50, 10.51) | 4.90(2.56, 6.67) | |

**Table 3. Analysis of progression-free survival of patients (PFS, month)**

| Stratification | | Group | Number of cases | Number of censored cases | Progression-free survival | *P* value |
|---|---|---|---|---|---|---|
| | | | | | P₅₀ | |
| Clinical stage before randomization | Limited stage | Test group | 10 | 5 | 4.83 | |
| | | Control group | 4 | 1 | 2.40 | |
| | Extended stage | Test group | 71 | 23 | 3.84 | <0.0001 |
| | | Control group | 34 | 4 | 0.72 | |
| Chemotherapy line | Second -line | Test group | 63 | 24 | 4.11 | <0.0001 |
| | | Control group | 28 | 3 | 0.72 | |
| Whether CR or PR was achieved with second -line chemotherapy | No | Test group | 69 | 24 | 4.11 | <0.0001 |
| | | Control group | 35 | 5 | 0.72 | |
| | Yes | Test group | 12 | 4 | 2.79 | |
| | | Control group | 3 | 0 | 1.48 | |
| Whether hasbeen treated with "irinotecan + platinum" | No | Test group | 12 | 4 | 2.83 | 0.0001 |
| | | Control group | 10 | 3 | 0.76 | |
| | Yes | Test group | 69 | 24 | 4.07 | <0.0001 |
| | | Control group | 28 | 2 | 0.72 | |
| Whether has been treated with "paclitaxel" | No | Test group | 69 | 27 | 4.11 | <0.0001 |
| | | Control group | 27 | 2 | 0.72 | |
| | Yes | Test group | 12 | 1 | 2.86 | 0.0160 |
| | | Control group | 11 | 3 | 0.76 | |
| Whether has been treated with "docetaxel" | No | Test group | 71 | 25 | 4.07 | <0.0001 |
| | | Control group | 29 | 3 | 0.72 | |
| | Yes | Test group | 10 | 3 | 2.83 | 0.0003 |
| | | Control group | 9 | 2 | 0.69 | |
| Whether brain metastasis upon enrollment | Yes | Test group | 21 | 6 | 3.84 | 0.0032 |
| | | Control group | 9 | 3 | 0.76 | |

**Table 4. Analysis of overall survival of patients (OS, month)**

| Stratification | | Group | Number of cases | Number of censored cases | Overall survival | *P* value |
|---|---|---|---|---|---|---|
| | | | | | P₅₀ | |
| Clinical stage before randomization | Extended stage | Test group | 71 | 42 | 8.05 | 0.0074 |
| | | Control group | 34 | 14 | 4.90 | |
| Chemotherapy line | Second -line | Test group | 63 | 40 | 7.29 | 0.0329 |
| | | Control group | 28 | 12 | 4.90 | |
| Whether CR or PR was achieved with second -line chemotherapy | No | Test group | 69 | 43 | 7.29 | 0.0093 |
| | | Control group | 35 | 16 | 4.90 | |
| | Yes | Test group | 12 | 6 | 6.80 | |
| | | Control group | 3 | 1 | 6.47 | |
| Whether brain metastasis upon enrollment | No | Test group | 60 | 42 | 8.80 | |
| | | Control group | 29 | 15 | 6.47 | |
| | Yes | Test group | 21 | 7 | 6.08 | 0.0061 |
| | | Control group | 9 | 2 | 2.56 | |

### Example 4

A female aged 49 was pathologically diagnosed with small cell lung cancer of the left lower lobe in October 2013 after surgery. On December 24, 2016, MRI showed multiple metastases in bilateral cerebral hemisphere, cerebellar hemisphere and brain stem, as well as multiple nodes with abnormal signal in liver. It was considered to be metastatic tumor, suggesting progressive disease. The patient was admitted to the hospital for one cycle of liposomal paclitaxel + carboplatin + temozolomide chemotherapy and was discharged on December 28.

One cycle of TC regimen (paclitaxel + carboplatin) was started on January 17, 2017, and the efficacy was evaluated as PD on February 10, 2017. One cycle of IP regimen (irinotecan 120 mg d1, 80 mg d8; cisplatin 100 mg d1) was started on February 16, 2017. On April 24, CT showed that the lesions in the lung and brain were stable, the liver metastasis was larger than before, and the efficacy was evaluated as PD; target lesion (64 mm): the left internal lobe node and the right posterior lobe node of the liver; non-target lesions: the multiple metastases in the brain.

On May 12, 2017, oral administration of a capsule of the dihydrochloride of the compound of formula I at 12 mg was started once daily for 2 weeks and interrupted for 1 week, every 3-week as one treatment cycle until PD or intolerance. Overall, the administration was well tolerated and could be continued.

The CT results were as follows:
After the first treatment cycle : target lesion: 63 mm; non-target lesions: non-CR/non-PD.
After the second treatment cycle : target lesion: 65 mm; non-target lesions: non-CR/non-PD.
After the fourth treatment cycle : target lesion: 66 mm; non-target lesions: non-CR/non-PD.
After the sixth treatment cycle : target lesion: 72 mm; non-target lesions: non-CR/non-PD.

## Claims

1. Use of a compound of formula I or a pharmaceutically acceptable salt thereof for the manufacture of a medicament for treating small cell lung cancer,

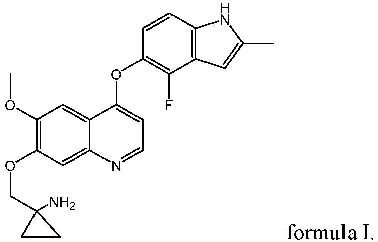

2. The use according to claim 1, wherein the small cell lung cancer is one that has failed with at least two chemotherapy regimens.

3. The use according to claim 1 or 2, wherein the small cell lung cancer is one that has failed with second-line and/or higher-line chemotherapy.

4. The use according to claim 2 or 3, wherein the chemotherapy is one or more of cisplatin, etoposide, carboplatin, nedaplatin, oxaliplatin, miriplatin, lobaplatin, irinotecan, topotecan, paclitaxel, docetaxel, temozolomide, vinorelbine, gemcitabine, cyclophosphamide, doxorubicin, vincristine, bendamustine, epirubicin, methotrexate and amrubicin.

5. The use according to any one of claims 1 to 4, wherein the small cell lung cancer is refractory and relapsed small cell lung cancer.

6. The use according to any one of claims 1 to 5, wherein the small cell lung cancer is extensive-stage small cell lung cancer.

7. The use according to any one of claims 1 to 6, wherein the small cell lung cancer is one with brain metastasis

8. The use according to any one of claims 1 to 7, wherein the pharmaceutically acceptable salt thereof is a salt formed by the compound of formula I and any of the following acids: hydrochloric acid, hydrobromic acid, sulfuric acid, nitric acid, phosphoric acid, acetic acid, trifluoroacetic acid, propionic acid, hexanoic acid, heptanoic acid, cyclopentane propionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, 1,2-ethanedisulfonic acid, 2-hydroxyethanesulfonic acid, benzenesulfonic acid, *p*-chlorobenzenesulfonic acid, *p*-toluenesulfonic acid, 3-phenylpropionic acid, trimethylacetic acid, *t*-butylacetic acid, dodecyl sulfuric acid, gluconic acid, glutamic acid, hydroxynaphthoic acid, salicylic acid, and stearic acid; preferably, the pharmaceutically acceptable salt thereof is a hydrochloride salt or maleate salt; and more preferably, the pharmaceutically acceptable salt thereof is a dihydrochloride salt.

9. A method for treating small cell lung cancer, comprising administering to a patient in need of treatment a therapeutically effective amount of a compound of formula I or a pharmaceutically acceptable salt thereof,

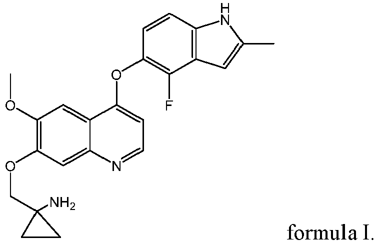

10. The method according to claim 9, wherein the small cell lung cancer is one that has failed with at least two chemotherapy regimens.

11. The method according to claim 9 or 10, wherein the small cell lung cancer is one that has failed with second-line and/or higher-line chemotherapy, and/or is refractory and relapsed small cell lung cancer.

12. The method according to claim 10 or 11, wherein the chemotherapy is one or more of cisplatin, etoposide, carboplatin, nedaplatin, oxaliplatin, miriplatin, lobaplatin, irinotecan, topotecan, paclitaxel, docetaxel, temozolomide, vinorelbine, gemcitabine, cyclophosphamide, doxorubicin, vincristine, bendamustine, epirubicin, methotrexate and amrubicin.

13. The method according to any one of claims 9 to 12, wherein the small cell lung cancer is extensive-stage small cell lung cancer and/or small cell lung cancer with brain metastasis.

14. The method according to any one of claims 9 to 13, wherein the pharmaceutically acceptable salt thereof is a salt formed by the compound of formula I and any of the following acids: hydrochloric acid, hydrobromic acid, sulfuric acid, nitric acid, phosphoric acid, acetic acid, trifluoroacetic acid, propionic acid, hexanoic acid, heptanoic acid, cyclopentane propionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, 1,2-ethanedisulfonic acid, 2-hydroxyethanesulfonic acid, benzenesulfonic acid, *p*-chlorobenzenesulfonic acid, *p*-toluenesulfonic acid, 3-phenylpropionic acid, trimethylacetic acid, *t*-butylacetic acid, dodecyl sulfuric acid, gluconic acid, glutamic acid, hydroxynaphthoic acid, salicylic acid, and stearic acid; preferably, the pharmaceutically acceptable salt thereof is a hydrochloride salt or maleate salt; and more preferably, the pharmaceutically acceptable salt thereof is a dihydrochloride salt.

15. The method according to any one of claims 9 to 14, wherein the compound of formula I or the pharmaceutically acceptable salt thereof is administered at a daily dose of 3 mg to 30 mg, preferably 5 mg to 20 mg, more preferably 8 mg to 16 mg, further more preferably 8 mg to 14 mg, and most preferably 8 mg, 10 mg, or 12 mg.

16. The method according to any one of claims 9 to 15, wherein the compound of formula I or the pharmaceutically acceptable salt thereof is administered in an intermittent regimen of treatment periods and interruption periods; the ratio of the treatment period to the interruption period in days is preferably 2:0.5-2:5, more preferably 2:0.5-2:3, even more preferably 2:0.5-2:2, and further more preferably 2:0.5-2:1; the intermittent regimen is further preferably in one of the following cycles: 2-week treatment plus 2-week interruption, 2-week treatment plus 1-week interruption, and 5-day treatment plus 2-day interruption.

17. A pharmaceutical composition for treating small cell lung cancer, comprising a compound of formula I or a pharmaceutically acceptable salt thereof, and at least one pharmaceutically acceptable carrier,

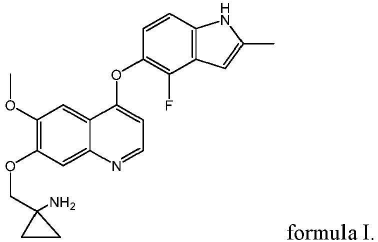

18. The pharmaceutical composition according to claim 17, wherein the small cell lung cancer is one that has failed with at least two chemotherapy regimens.

19. The pharmaceutical composition according to claim 17 or 18, wherein the small cell lung cancer is one that has failed with second-line and/or higher-line chemotherapy, and/or is refractory and relapsed small cell lung cancer.

20. A kit comprising (a) at least one unit dose of the pharmaceutical composition according to any one of claims 17 to 19 and (b) instruction for treating small cell lung cancer, in particular instruction t for treating small cell lung cancer in an intermittent regimen, wherein the pharmaceutical composition is preferably a preparation suitable for oral administration, more preferably a tablet or capsule.
